# EUROPEAN PATENT SPECIFICATION

(11) **EP 4 584 794 B1**
(45) Date of publication and mention of the grant of the patent: **05.11.2025**
(21) Application number: 23764643.5
(22) Date of filing: 04.09.2023
(51) Int. Cl.: G16H 20/40, A61C 7/00, G16H 50/20, G06N 20/00, A61C 13/00, A61C 19/04

(54) **DENTAL TREATMENT PAIN PREDICTION**
SCHMERZVORHERSAGE FÜR ZAHNBEHANDLUNGEN
PRÉDICTION DE LA DOULEUR D'UN TRAITEMENT DENTAIRE

(30) Priority: 05.09.2022 EP 22193836
(43) Date of publication of application: 16.07.2025
(73) Proprietor: Koninklijke Philips N.V., 5656 AG Eindhoven (NL)
(72) Inventor: SPELT, Hanne Adriana Alijda, 5656 AG Eindhoven (NL); KOOIJMAN, Gerben, 5656 AG Eindhoven (NL); JOHNSON, Mark Thomas, 5656 AG Eindhoven (NL)
(74) Representative: Philips Intellectual Property & Standards
(86) International application number: PCT/EP2023/074101
(87) International publication number: WO 2024/052244

(56) References cited:
- EP-A1- 2 987 451
- EP-B1- 2 164 422
- US-A1- 2008 306 724
- US-A1- 2022 223 293

## Description

### FIELD OF THE INVENTION

The present invention relates to the field of oral health care, particularly in the clinical context.

### BACKGROUND OF THE INVENTION

Anticipated treatment discomfort or pain is a significant barrier to patient compliance with regular consultation and treatment with dental practitioners. Treatment pain can cause a patient to develop dental anxiety. Experience of pain at a previous dental visit has been shown to be correlated with dental anxiety. An estimated 3-16% of adults suffer from dental phobia. Patient studies indicate that patients believe that dental practitioners (DPs) should pay attention to any discomfort (e.g., pain, stress, or anxiety) during treatment and that DPs should take steps to ameliorate this. However, anticipated treatment discomfort is not a major cause of anxiety for the whole patient population. In a study in which the sample was representative of the general population aged 15 years and over, 60% reported having at least one dental experience involving 'strong pain', of which only 5-6% experienced dental treatment in general to be very painful.

In addition to affecting patients, treatment pain and anxiety are also an issue affecting dental practitioners. It has been found in surveys that 60% of DPs perceive dentistry as more stressful than other professions. Among the top 5 most significant stressors were 'causing pain' (second place) and 'anxious patient' (fifth place).

Thus, it is a mutually beneficial outcome if dental treatment steps can be applied in a way that minimizes patient pain, thereby reducing anxiety about future treatment.

Dental practitioners typically seek to do this as part of their standard practice, by anticipating steps which are likely to cause more pain than others and giving clear warnings and reassurances to a patient, and taking extra care or working more slowly during these steps. For example, particularly more experienced DPs are able to carry out a single treatment with multiple different treatment styles or philosophies, and are able to vary the treatment style according to the pain experienced by the patient in each step, e.g., speed up treatment steps that inflict less pain and take a careful approach when pain is expected.

However, it is difficult in practice to accurately estimate the amount of pain associated with particular steps of a treatment, and with the particular style applied for that step. The pain experienced can also vary depending upon the pain threshold of the patient. Dentistry physically limits the patient's ability to communicate verbally during the treatment phase of a consultation. These ineffective communications can lead to misunderstanding about the pain which a patient may be experiencing. Many dental practitioners will ask a patient to raise their hand if they experience (too much) pain. However, when the patient raises their hand, he/she is already in pain. Furthermore, there might be a delay of some seconds between the patient indicating the pain and the dental practitioner adapting their treatment style to ameliorate this. Additionally, a DP typically has no insight into whether the patient is comfortable and thus whether a particular treatment step might be made more intense.

EP 2 164 422 discloses systems of managing planning and delivery of an orthodontic treatment using planning tools, treatment guidelines, instructions and appointment planning tools customized to the individual patient being treated, as well as tools for tracking delivery and patient progression through an orthodontic treatment plan. US 2022/223293 focuses on predictive medical diagnosis, especially for use in screening, early detection, and treatment selection of autoimmune-related conditions. EP 2 987 451 relates to a methodology and tool for predicting a pain response for a general treatment of a subject

### SUMMARY OF THE INVENTION

In view of the above, it is the recognition of the inventors that it would be beneficial to provide dental practitioners (DPs) with means for determining with greater clarity expected treatment pain for a given patient during each step of a particular treatment. These insights on treatment pain can help guide a dental practitioner in adapting their style or approach to each step to mitigate expected pain.

Furthermore, the inventors have realized that it would be beneficial for a DP to be able to estimate the expected treatment discomfort for a particular step in the procedure also taking account of dentist's treatment style (i.e., the approach or attitude of the DP towards conducting certain treatments). With this knowledge, the DP could e.g., speed up treatment steps that inflict less pain, while taking a more careful approach when pain is expected. This way, dentists can adapt their treatment style to the needs of the patient.

The invention is defined by the independent claims, while other advantageous embodiments are defined by the dependent claims.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components and a processing flow of an example processing arrangement in accordance with one or more embodiments of the invention;
Fig. 3 schematically illustrates processing flow in accordance with one or more embodiments of the invention; and
Fig. 4 schematically illustrates processing flow in accordance with a further one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for generating prediction information comprising a predicted measure of treatment pain associated with each of a series of steps to be performed as part of a dental treatment by a dental practitioner. For example, a machine learning algorithm could be used, which is trained on historical pain measurement information for the same type of treatment. This allows the algorithm to learn from the historical pain associated with different steps. This information can be output as guidance for the practitioner, allowing them to adapt their treatment style or approach for each step to pre-emptively mitigate expected pain.

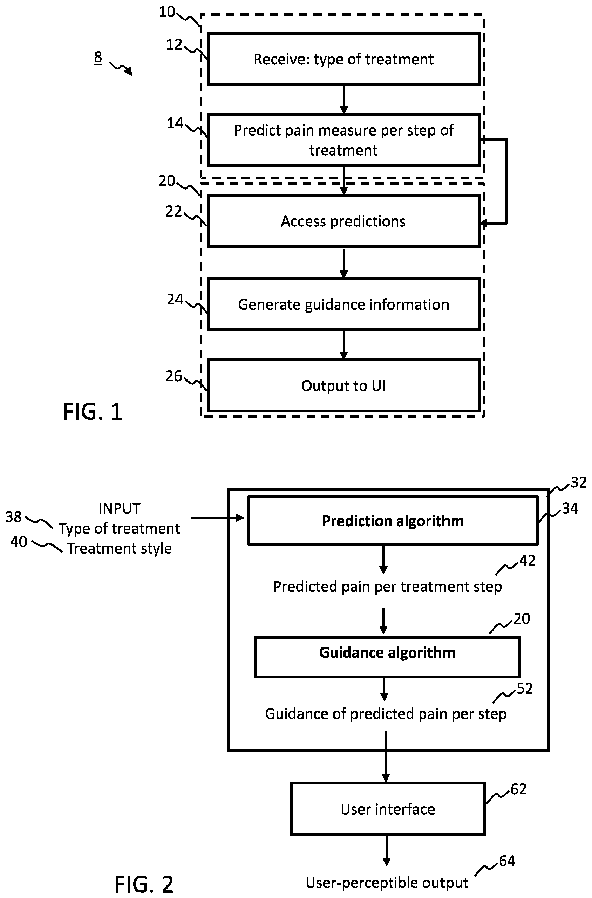

Fig. 1 outlines in block diagram form steps of an example method according to one or more embodiments. The steps will be recited in summary, before being explained further in the form of example embodiments.

The method 8 is a computer-implemented method for use in providing guidance to a dental practitioner in respect of a dental treatment.

The method 8 comprises running a prediction algorithm 10. The prediction algorithm is configured to receive 12 input variables comprising at least: a type of treatment required by the patient, for example selected from a pre-defined set of possible treatment types. The prediction algorithm is further configured to generate 14 an output prediction dataset comprising a predicted measure of treatment pain for each of a series of steps comprised by the input type of treatment.

The inputs received by the prediction algorithm may further include a treatment style to be implemented by the dental practitioner, for example selected from a pre-defined set of possible treatment styles.

In some embodiments, the method 8 may further comprise running a guidance algorithm, the guidance algorithm 20. The guidance algorithm may be configured to: access 22 the prediction dataset; generate 24 guidance information indicative of the predicted treatment pain for at least a subset of the series of treatment steps; and control 26 a user interface to generate at least one user-perceptible output indicative of the guidance information.

In some embodiments, the prediction algorithm comprises at least one trained machine learning algorithm.

The method might for example comprise controlling 26 the user interface to generate an ordered sequence of user perceptible outputs, each indicative of the predicted treatment pain for one of the series of treatment steps. The generation of each next user-perceptible output could be triggered by a user input from the user interface, or is triggered according to a pre-defined timing schedule. The timing schedule could be pre-defined in advance of execution of the guidance algorithm based on a user input.

In some embodiments, the input variables to the prediction algorithm further include one or more patient oral health characteristics.

The prediction algorithm may be pre-trained in this case to generate predicted pain tailored to the particular one or more health characteristics. One illustrative example for instance is whether the patient is prone to gingivitis, in which case, more pain might be predicted. Other examples of oral health characteristics may include, by way of illustration: presence or absence of sensitive gums, presence of an infection, recent intervention history (e.g. extraction, pocket treatment), presence of an abscess or damage to the root of a tooth, or jaw problems (e.g. TMJ - pain opening the jaw).

In some advantageous embodiments, the method may further comprise accessing a scheduling database storing records indicative of scheduled treatment sessions, each record including at least an indication of a type of treatment to be performed. This step results in obtaining information of the treatment type which is to be administered for a particular patient. For example, the scheduling database might be queried using patient identification information, to obtain the scheduling record for a relevant patient, and from this obtain the indication of the treatment type.

Optionally, the method may further comprise receiving information indicative of a treatment style to be implemented by the dental practitioner. For example this might be input by the dental practitioner to a user input device, or the treatment style to be implemented could also be comprised as part of each data record in the scheduling database, and be obtained from this record.

The method may comprise generating the input variables for the prediction algorithm based on said accessing and said receiving steps.

Thus, this automates the process of obtaining the needed inputs to the prediction algorithm.

Although in the description above, the various steps of the method are designated as being performed by specific algorithms, it will be appreciated that in other examples, the steps of the method may all be performed by a single algorithm, or by more than two algorithms. The inventive concept is not limited according to any particular division of steps between particular algorithms. The designation is for convenience of description and reflects a most likely implementation in practice.

Reference has been made to the option of the prediction algorithm taking into account treatment style in generating the prediction of the pain measure for each treatment step. By way of example, the treatment styles could include one or more of: slow, careful approach; more rapid, forceful approach; mechanical approach; pharmaceutical approach. Another example is a minimal anesthesia approach in which anesthesia use is minimized. Another approach is a precautionary antibiotics approach in which antibiotics are administered after any dental treatment, on a 'just in case' basis. Another approach is a minimal antibiotics approach in which antibiotics are administered only if essential. Another approach is a fast/flexible approach in which a practitioner, upon performing dental examination will give the option to perform the needed treatment straight away (even if this may be more painful that waiting for an appointment some weeks later for which preparations could be made for anesthesia). Another approach is a precautionary treatment approach in which superficial caries would be treated with mechanical (drilling) treatment. Another approach (opposite to the precautionary treatment approach) would be the minimal treatment approach in which a response to superficial caries would be to prescribe a high fluoride toothpaste, and/or to administer a fluoride rinse, and to schedule monitoring appointments.

With respect to the method of this invention, the pre-defined set of treatment styles can be defined in whatever way is desired. Each would be given a label or name, and a definition of it (in terms of its characteristics) would be defined in advance. This would be known to the dental practitioner. Then, when providing treatment style information for input to the prediction algorithm, the dental practitioner could use the known definitions to decide what style of the list best matches his or her own style.

Likewise, when compiling the training database used to train the optional machine learning algorithm of the prediction algorithm, the treatment providers who contribute data to this database would also be provided the list of pre-defined treatment styles with its definitions of each style, and in this way they could label or tag each treatment session data entry with the corresponding style that best matches the style that was used for that treatment.

Following the above, there is consistency of definition across all parts of the method and system, including all databases and algorithms, in terms of the definitions of the treatment styles.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention, and also schematically showing the processing flow in more detail. The processing unit 32 comprises one or more processors (not shown) and may comprise an input/output. The one or more processors are adapted to run a prediction algorithm 34, and the prediction algorithm configured to: receive input variables comprising at least: a type of treatment 38 required by the patient, for example selected from a pre-defined set of possible treatment types. Optionally, the input variables may further include at least a treatment style 40 to be implemented by the dental practitioner. The prediction algorithm 34 is further configured to generate an output prediction dataset comprising a predicted measure of treatment pain 42 for each of a series of steps comprised by the input type of treatment. The prediction algorithm may comprise at least one trained machine learning algorithm.

In some embodiments, the one or more processors of the processing unit 32 may be further adapted to run a guidance algorithm 20, the guidance algorithm configured to access the prediction dataset, and generate guidance information 52 indicative of the predicted treatment pain for at least a subset of the series of treatment steps, and control a user interface 62 to generate at least one user-perceptible output 64 indicative of the guidance information.

For example, the user interface 62 may comprise a display device for generating visual user-perceptible outputs. This may for example be a display device connected with a computing device located for example in the treatment room used by the dental practitioner. In this way the dental practitioner can be provided the guidance information during the treatment. Alternatively, the user interface could be located elsewhere, for example enabling the dental practitioner to review the guidance in advance of the treatment.

The processing unit 32 could be part of a computing device of the dental practitioner, for example a mobile computing device, for example a smartphone or tablet. It could be part of a dental device in other examples. It could be part of a cloud-based server in some examples. It could be part of a computing workstation in some examples.

By way of illustration, an example implementation of the method according to a particular set of embodiments considered particularly advantageous by the inventors, could be summarized as comprising the following features:
a processor which is configured to execute an algorithm that is programmed to:
- receive information on the upcoming consultation, which includes the type of treatment and the treatment style/philosophy, and optionally patient characteristics;
- predict the expected treatment pain for each treatment step; and
- provide guidance or training to the dental practitioner by generating feedback indicative of the expected pain level or amount of each treatment step during treatment.

As noted above, a part of the method may include the use of a prediction algorithm in the form of a trained machine learning algorithm. Details in respect of the trained machine learning algorithm will now be discussed in further detail.

A machine-learning algorithm is any self-training algorithm that processes input data in order to produce or predict output data. In the present case, the input data comprises, at minimum, the treatment type required by the patient, and optionally also a treatment style to be implemented by the dental practitioner, and the output data comprises a predicted measure of treatment pain for each of a series of steps comprised by the input type of treatment.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines, Naive Bayesian models, and k-nearest neighbor models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

In the present case, the training dataset may be based on data contained in a database which comprises respective data entries for each of a plurality of prior/historical treatment sessions by a plurality of different patients to a set of different dental treatment providers, and wherein each treatment session entry includes at least an indication of:
- a type of treatment administered;
- a measure indicative of treatment pain for each of a series of steps comprised by the treatment
and preferably also an indication of:
- a treatment style, where the treatment style is one of the said pre-defined set of treatment styles.

In this case, the training input data entries of the training data may correspond to the type of treatment needed by the patient and optionally the treatment style. The training output data entries may correspond to a measure indicative of predicted pain for each of a series of steps of the treatment type.

Another alternative would be to use a prediction algorithm which comprises a plurality of trained machine learning algorithms, wherein each has been trained using a filtered subset of the training dataset comprising only those training data entries which correspond to a particular respective one of the treatment styles. In this case, the training input data for each of the machine learning algorithms, may comprise just the type of treatment (at minimum) and the training output data entries may comprise (at minimum) just the predicted measure of treatment pain. Then, when running the prediction algorithm in the context of the present invention, the algorithm may use the input variable indicative of the treatment style to be implemented by the dental practitioner to select the particular one of the plurality of trained machine learning algorithms which has been trained to generate pain predictions for the particular input treatment style. This machine learning algorithm may then be run by the prediction algorithm with an input comprising the type of treatment needed by the patient, and wherein the algorithm generates an output indicative of a predicted measure of pain for each of a series of steps of the treatment for the particular treatment style which indicated by the input variable.

In some embodiments, at least a subset of the treatment session data entries in the training database may further include an indication of one or more of: a treatment duration; a treatment cost; one or more oral health characteristics of the patient; a geographical location of the treatment provider; and/or one or more patient personality characteristics; and wherein the machine learning algorithm is trained accordingly.

In some embodiments, the measure indicative of treatment pain for each of the series of steps included in the data entries is a quantitative measure. For example, it may correspond to a measured total pain amount. By way of example, the total measured pain amount may correspond to a product of a measured pain response level and a time for which the pain response level was experienced, for each of one or more pain response episodes. Alternatively, the measure indicative of treatment pain for each of the series of steps may correspond to a maximum or peak pain level experienced during the respective treatment step. In still further embodiments the measure indicative of treatment pain for each of the series of steps may correspond to a rate of change in the pain level experienced during the respective treatment step, or a maximum rate of change in the pain level experienced during the treatment step.

For example, the pain information can be derived from a biological sensing signal acquired as a function of time during the treatment in the plurality of historical treatment sessions.

By way of one example, during a (historical) treatment session, an objective measure of treatment pain can be derived in the following way, for generating the data entry for the database.

A processor at the treatment provider office can be adapted for classifying a subject's response to a series of contact events during a measurement session, the processor adapted to: receive, during the measurement session, an indication of timings of a series of oral contact events, each contact event corresponding to mechanical engagement of an oral tool with a surface within the oral cavity of a subject; receive, during the measurement session, a biological signal for the subject from a biological signal sensing apparatus coupled to the subject; identify physiological response events in the biological signal; determine a temporal proximity between each physiological response event and an immediately preceding contact event, and classify a subject pain and/or non-pain arousal associated with the physiological response event based thereon. A physiological response event may take the form of a fluctuation or oscillation in the signal, e.g. a peak or drop in the signal. For example, the processor may be configured to classify a physiological response event as associated with a pain state dependent on the physiological response event occurring within a pre-defined time window immediately following a contact event. The classification can further comprise determining a pain level of the pain state based on one or more properties of the physiological response event peak or drop, and preferably wherein the one or more properties include a maximum height of a leading edge of the peak or drop. The classification can be performed in real time during the measurement session, and for each individual epoch corresponding to each respective step of the treatment.

The processor can be adapted to compute a total pain amount over the time periods spanned by each respective treatment step, the total pain amount being a product of a pain level and a time for which the pain was experienced, the pain level being computed for each physiological response event peak or drop having a pain classification based on one or more properties of the physiological response event peak or drop. The processor can also be configured to classify a physiological response event as associated with a non-pain arousal state dependent on the physiological response event occurring outside of a time window of pre-defined duration following any contact event. The processor could optionally be adapted to further compute a total non-pain arousal amount over the time period spanned by each treatment step, the total non-pain arousal amount being a product of an arousal level and a time for which the arousal was experienced, the arousal level being computed for each physiological response event peak or drop having a non-pain arousal classification based on one or more properties of the physiological response event peak or drop.

Within the office of the treatment provider, there could be a system comprising: a contact identification means for identifying timings of a series of oral contact events, each contact event corresponding to mechanical engagement of an oral tool with a surface within the oral cavity of a subject; a biological signal sensing apparatus for measuring a physiological response of the subject (such as a skin conductance sensor or heart rate sensor or any other biosignal sensor); and a processor as already described, arranged to receive, over a measurement session, one or more contact signals from the contact identification means indicative of timings of the series of oral contact events and a biological signal from the biological signal sensing apparatus.

Thus, it can be recognized how an objective measure of a pain associated with each step of a particular treatment can be acquired based on biosignals. The measure of treatment pain recorded in the database used to train the machine learning algorithm could, for each database treatment session entry, correspond to the total pain amount measured over each individual treatment step, or could correspond to the highest experienced pain level during each treatment step, or could correspond to an average pain level throughout the time period covered by each treatment step, or a ratio of total pain to total non pain amount during each treatment step, or any other variable or parameter derived from the biosignal pain data discussed above acquired during each historical treatment session and for each treatment step.

This information could then be stored as an entry in the database which is used to train the prediction algorithm 34, along with indication of the type of treatment administered, and optionally a treatment style, where the treatment style is one of the said pre-defined set of treatment styles.

Indeed, another aspect of this invention may be the compilation of the training dataset referred to above, using the pain measurement technique mentioned above, or another objective pain measurement technique.

Another aspect of the invention may be the training of the prediction algorithm in accordance with the example method already described above.

An aspect of the invention could include a combination of the compilation of the training database using the pain measurement technique described above and the training of the prediction algorithm using this training database in accordance with the example method described above.

An embodiment of the invention may comprise:
the compilation of the training database using for example one or more features of the pain measurement technique described above, or another technique;
the training of the machine learning algorithm comprised by the prediction algorithm using this training database; and
the execution of the pain prediction method steps and optionally guidance generation method steps already recited above with reference to Fig. 1 (or any other embodiment / example of this method outlined in this document, or in accordance with any claim of this application).

An example implementation of a method according to a particular set of embodiments of the invention will now be described by way of illustration of the concept of the invention summarized in Fig. 1. It will be appreciated that not all features of this particular set of embodiments are essential to the inventive concept, and are described to aid understanding and to provide an example to illustrate the inventive concepts.

In accordance with this particular set of embodiments a system and method is provided which is configured for providing feedback or training information for a dental practitioner during a treatment part of a patient consultation. As has already been discussed, to predict the expected treatment pain, an algorithm is trained using a large patient database resulting, for example, from objective pain measurements recorded using the methods already discussed, based on measured biological signals during historical treatment sessions.

This training database might for example include for each case at least information on: a type of treatment (e.g. cleaning), a style or philosophy of treatment (e.g. mechanical, careful approach, forceful approach), a measure of the experienced treatment pain, preferably during each step of the treatment (e.g. visual analogue scales, or skin responses), and optionally: treatment time, treatment costs, and/or patient oral health characteristics (e.g. prone to gingivitis).

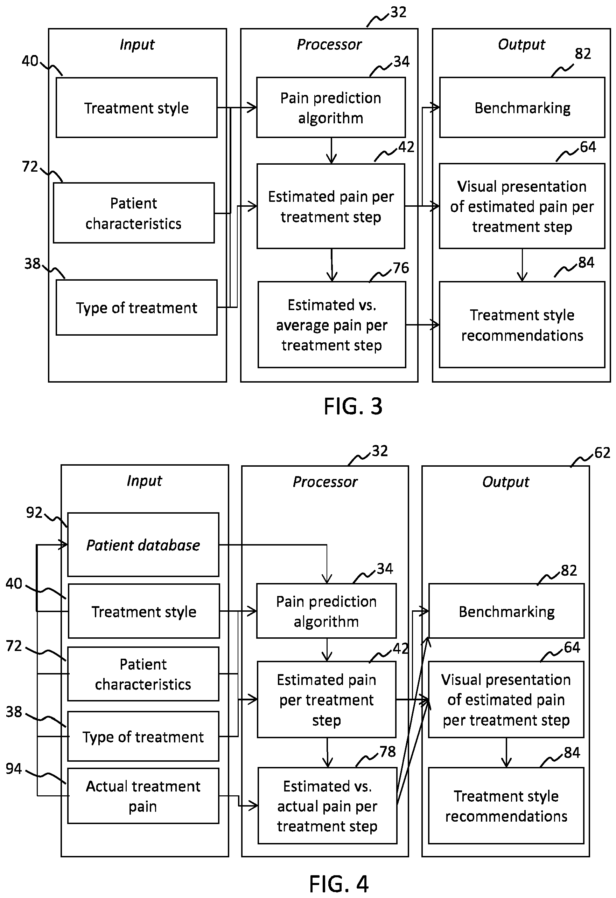

The processing flow according to this particular set of embodiments is outlined schematically in Fig. 3.

The algorithm receives information on the type of treatment 38 (e.g. from a dental records or scheduling system or database of the dental practitioner). The algorithm also receives an indication of a treatment style or philosophy of the dental practitioner (e.g. self-reported upfront or measured in real-time). Optionally the algorithm may also receive information indicative of one or more patient oral health characteristics 72.

A pain prediction algorithm 34, embodied in a processor 32, is configured to predict the expected treatment pain 42 of each step in the procedure, preferably taking into account the treatment style of the dental practitioner.

These estimated pain levels per treatment step are then presented visually 64 to the dental practitioner, for guidance or for personalized training purposes (e.g. by showing a pain prediction score per step).

Based on this output 64, the dental practitioner could for example choose to adapt his or her treatment by, e.g. speeding up parts that are not predicted to cause a lot of discomfort and being careful during parts that are likely to cause discomfort.

Especially more experienced dental practitioners have the skill to perform a certain treatment with different styles and approaches, e.g. fast (and therefore cheaper), but more painful, versus slower and less painful, but more costly.

As discussed above, optionally, the processor 32 is further configured to compare 76 the expected treatment pain, preferably considering the treatment style of the dental practitioner, with the average treatment pain for similar treatment types, across all styles, and feeds this information back to the dental practitioner.

Optionally, the processor may generate and output recommendations 84 to adapt the treatment style to mitigate pain, such as: suggesting an alternative treatment style, scheduling an appointment for the treatment with more or less time, adding or adapting a dose of anesthesia, suggesting a greater number or fewer support staff, and/or providing specific warnings or instructions to the patient up-front.

Optionally, the output predicted treatment pain may be used to benchmark 82 the treatment style and skill of different staff members within one dental practice or between dental practices - thereby improving the quality of care.

According to an extension of the above-described example, in some embodiments, additionally a means may be provided to measure the actual treatment pain (e.g. visual analogue scales, or through measurement of a biological signal such as electrodermal activity).

An extended processing flow according to this particular set of embodiments is outlined schematically in Fig. 4.

During the treatment, the patient's pain levels 94 are assessed at each (or at least a subset) of the steps of the treatment. Then, either after or during the treatment or consultation, the processor 32 is configured to compare 78 the actual pain levels 94 to the estimated pain levels 42. This comparison may be fed back as output information to the dental practitioner. They may be output through visual presentation 64 by user an user interface for example. This information may function for example as personalized training for the dental practitioner.

Additionally or alternatively, the comparisons 78 between estimated 42 and actual treatment pain 94 per step can be used for benchmarking purposes 82. Also, the actual 94 treatment pain, together with the other characteristics of the treatment can be used to update the training database 92 which is used to train the prediction algorithm 34. The prediction algorithm may be recurrently re-trained using the recurrently updated training database 92.

This additional feature of comparing actual with predicted pain for each step can indeed be applied as a more general concept to any embodiment of this invention.

Thus, stated in more general terms, the method of any embodiment might comprise steps of: obtaining a measure indicative of estimated actual treatment pain for at least a subset of the steps of the treatment; comparing for each of the at least subset of steps the estimated actual treatment pain and the predicted treatment pain; and generating feedback information for the dental practitioner based on the comparison, e.g. indicative of a result of the comparison. In preferred cases, the measure indicative of estimated actual treatment pain for each of said at least subset of steps of the treatment can be received in real time during performance of each of the subset of the steps of the treatment by the dental practitioner. Optionally, the comparison and feedback generation are performed in real time with receipt of the estimated actual treatment pain for each step. This allows the dentist to adapt their approach for each step in real time.

With regards to means for obtaining the measure indicative of treatment pain, technical means for doing this have indeed already been described earlier in this document, and the reader is referred to this for fuller details. By way of brief summary, the measure indicative of estimated actual treatment pain for at least a subset of the steps of the treatment may comprise: receiving, during each of the at least subset of steps of the treatment, a biological signal for the subject from a biological signal sensing apparatus coupled to the subject; identifying physiological response events in the biological signal; and determining the measure indicative of estimated actual treatment pain for each said step of the treatment based on the physiological response events identified for that step.

As a further feature of any embodiment, means may optionally be provided for measuring the treatment style of the dental practitioner in real-time (e.g. through pressure and movement sensors integrated in the treatment tools). Thereby, the predictions as to pain levels per step can optionally be updated based on changes in the treatment style in real-time.

In more detail, the method may further comprise in some embodiments: receiving data indicative of motion and/or force patterns of a dental tool during at least one step of the treatment; and applying a treatment style analyzer algorithm configured to estimate based on the motion and/or force patterns a treatment style associated therewith. This process may be performed for each step of the treatment. The treatment style analyzer algorithm could be a machine learning algorithm trained to associate particular patterns of force and/or motion with particular treatment styles, or it could be a rule-based algorithm which applies pre-define criteria, such as threshold ranges for different force/motion parameters, to classify the treatment style.

In some embodiments, the method may further comprise re-running the prediction algorithm using the treatment style estimated by the treatment style analyzer algorithm to generate a new prediction of the measure of treatment pain for the at least one step; and generating a user-perceptible output indicative of the new prediction.

In some embodiments, the pain prediction algorithm, or another algorithm employed by the method and processing unit, may be adapted to have communicative access to the database 92 which is used to train the prediction algorithm. The method may further comprise relating information about an upcoming scheduled treatment session to similar cases in the training database, e.g. based on treatment type, treatment style, and/or patient oral health characteristics. This may be done by querying the database to find such similar previous cases. For example a nearest neighbor approach might be employed. Based on the similar particular cases identified from the database, an expected treatment pain for different steps of the upcoming treatment might be predicted based on the measures of pain recorded in the identified previous cases. This thus differs in the prediction approach discussed so far, in that, instead of or in addition to using the trained machine learning algorithm to generate predictions which rely on whole population based learning, simple direct querying of the training database itself is used to find similar cases and make pain predictions based on this.

As mentioned previously, the invention can be embodied in software form. Thus another aspect of the invention is a computer program product comprising code means configured, when run on a processor, to cause the processor to perform a method in accordance with example or embodiment of the invention described in this document, or in accordance with any claim of this patent application.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit may include a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (8) for use in providing guidance to a dental practitioner in respect of a dental treatment, the method comprising:
running a prediction algorithm (10), the prediction algorithm comprising at least one trained machine learning algorithm, wherein the prediction algorithm is configured to:
receive (12) input variables comprising at least: a type of treatment required by the patient, selected from a pre-defined set of possible treatment types;
generate (14) an output prediction dataset comprising a predicted measure of treatment pain for each of a series of steps comprised by the input type of treatment; and
running a guidance algorithm (20), the guidance algorithm configured to:
access (22) the prediction dataset;
generate (24) guidance information indicative of the predicted treatment pain for at least a subset of the series of treatment steps;
control (26) a user interface to generate at least one user-perceptible output indicative of the guidance information;
wherein the prediction algorithm comprises a machine learning algorithm pre-trained using a training database comprising respective data entries for each of a plurality of prior visits by a plurality of different patients to a set of different dental treatment providers, and wherein each visit entry includes at least an indication of:
- a type of treatment administered; and
- a measure indicative of treatment pain for each of a series of steps comprised by the treatment.

2. The method of claim 1, wherein the prediction algorithm is configured to receive input variables which further include: a treatment style to be implemented by the dental practitioner, selected from a pre-defined set of possible treatment styles.

3. The method of claim 1 or 2, the method comprising controlling the user interface to generate an ordered sequence of user perceptible outputs, each indicative of the predicted treatment pain for one of the series of treatment steps.

4. The method of any of claims 1-3, further comprising:
accessing a scheduling database storing records indicative of scheduled treatment sessions, each record including at least an indication of a type of treatment to be performed;
receiving information indicative of a treatment style to be implemented by the dental practitioner, and optionally wherein the treatment style to be implemented is also comprised as part of each data record in the scheduling database;
generating the input variables for the prediction algorithm based on said accessing and said receiving steps.

5. The method of claim 2 or any of claims 3-4 when dependent on claim 2, wherein each visit entry of the training database further includes an indication of:
- a treatment style, where the treatment style is one of the said pre-defined set of treatment styles.

6. The method of any of claims 1-5 ,
wherein, within each visit entry, the measure indicative of treatment pain for each of the series of steps corresponds to a total measured pain amount, the total measured pain amount corresponding to a product of a measured pain response level and a time for which the pain response level was experienced, for each of one or more pain response episodes of each treatment step;
wherein, within each visit entry, the measure indicative of treatment pain for each of the series of steps comprises a maximum or peak pain level experienced during the respective treatment step; and/or
wherein, within each visit entry, the measure indicative of treatment pain for each of the series of steps comprises a measure of a maximum rate of change of pain level experienced during each of the treatment steps.

7. The method of any of claims 1-6, further comprising:
obtaining a measure indicative of estimated actual treatment pain for at least a subset of the steps of the treatment;
comparing for each of the at least subset of steps the estimated actual treatment pain and the predicted treatment pain;
generating feedback information for the dental practitioner based on the comparison, e.g. indicative of a result of the comparison.

8. The method of claim 7, wherein the measure indicative of estimated actual treatment pain for each of said at least subset of steps of the treatment is received in real time during performance of each of the subset of the steps of the treatment by the dental practitioner, and wherein the comparison and feedback generation are generated in real time with receipt of the estimated actual treatment pain for each step.

9. The method of claim 7 or 8, wherein the obtaining a measure indicative of estimated actual treatment pain for at least a subset of the steps of the treatment comprises:
receiving, during each of the at least subset of steps of the treatment, a biological signal for the subject from a biological signal sensing apparatus coupled to the subject;
identifying physiological response events in the biological signal;
determining the measure indicative of estimated actual treatment pain for each said step of the treatment based on the physiological response events identified for that step.

10. The method of any of claims 1-9, further comprising:
receiving data indicative of motion and/or force patterns of a dental tool during at least one step of the treatment; and
applying a treatment style analyzer algorithm configured to estimate based on the motion and/or force patterns a treatment style associated therewith.

11. The method of claim 10, further comprising
re-running the prediction algorithm using the treatment style estimated by the treatment style analyzer algorithm to generate a new prediction of the measure of treatment pain for the at least one step; and
generating a user-perceptible output indicative of the new prediction.

12. A computer program product comprising code means configured, when run on a processor to cause the processor to perform a method in accordance with any of claims 1-11.

13. A processing unit (32), comprising:
an input/output; and
one or more processors, adapted to:
run a prediction algorithm (34), the prediction algorithm configured to:
receive input variables comprising at least: a type of treatment required by the patient (38), selected from a pre-defined set of possible treatment types, and
generate an output prediction dataset (42) comprising a predicted measure of treatment pain for each of a series of steps comprised by the input type of treatment; and
run a guidance algorithm (20), the guidance algorithm configured to:
access the prediction dataset;
generate guidance information (52) indicative of the predicted treatment pain for at least a subset of the series of treatment steps;
control a user interface (62) to generate at least one user-perceptible output (64) indicative of the guidance information;
wherein the prediction algorithm comprises a machine learning algorithm pre-trained using a training database comprising respective data entries for each of a plurality of prior visits by a plurality of different patients to a set of different dental treatment providers, and wherein each visit entry includes at least an indication of:
- a type of treatment administered; and
- a measure indicative of treatment pain for each of a series of steps comprised by the treatment.

14. The processing unit of claim 13, wherein the prediction algorithm is configured to receive input variables which further include: a treatment style to be implemented by the dental practitioner, selected from a pre-defined set of possible treatment styles.

15. The processing unit of claim 13 or 14, the one or more processors further adapted to control the user interface to generate an ordered sequence of user perceptible outputs, each indicative of the predicted treatment pain for one of the series of treatment steps.

16. The processing unit of any of claims 13-15, the one or more processors further adapted to:
access a scheduling database storing records indicative of scheduled treatment sessions, each record including at least an indication of a type of treatment to be performed;
receive information indicative of a treatment style to be implemented by the dental practitioner, and optionally wherein the treatment style to be implemented is also comprised as part of each data record in the scheduling database;
generate the input variables for the prediction algorithm based on said accessing and said receiving steps.

17. The processing unit of claim 14 or any of claims 15-16 when dependent on claim 14, wherein each visit entry of the training database further includes an indication of:
- a treatment style, where the treatment style is one of the said pre-defined set of treatment styles.

18. The processing unit of any of claims 13-17,
wherein, within each visit entry, the measure indicative of treatment pain for each of the series of steps corresponds to a total measured pain amount, the total measured pain amount corresponding to a product of a measured pain response level and a time for which the pain response level was experienced, for each of one or more pain response episodes of each treatment step;
wherein, within each visit entry, the measure indicative of treatment pain for each of the series of steps comprises a maximum or peak pain level experienced during the respective treatment step; and/or
wherein, within each visit entry, the measure indicative of treatment pain for each of the series of steps comprises a measure of a maximum rate of change of pain level experienced during each of the treatment steps.

19. The processing unit of any of claims 13-18, wherein the one or more processors are further adapted to:
obtain a measure indicative of estimated actual treatment pain for at least a subset of the steps of the treatment;
compare for each of the at least subset of steps the estimated actual treatment pain and the predicted treatment pain;
generate feedback information for the dental practitioner based on the comparison, e.g. indicative of a result of the comparison.

20. The processing unit of claim 19, wherein the measure indicative of estimated actual treatment pain for each of said at least subset of steps of the treatment is received in real time during performance of each of the subset of the steps of the treatment by the dental practitioner, and wherein the comparison and feedback generation are generated in real time with receipt of the estimated actual treatment pain for each step.

21. The processing unit of claim 19 or 20, wherein the obtaining a measure indicative of estimated actual treatment pain for at least a subset of the steps of the treatment comprises:
receiving, during each of the at least subset of steps of the treatment, a biological signal for the subject from a biological signal sensing apparatus coupled to the subject;
identifying physiological response events in the biological signal;
determining the measure indicative of estimated actual treatment pain for each said step of the treatment based on the physiological response events identified for that step.

22. The processing unit of any of claims 13-21, the one or more processors further adapted to:
receive data indicative of motion and/or force patterns of a dental tool during at least one step of the treatment; and
apply a treatment style analyzer algorithm configured to estimate based on the motion and/or force patterns a treatment style associated therewith.

23. The processing unit of claim 22, the one or more processors further adapted to:
re-run the prediction algorithm using the treatment style estimated by the treatment style analyzer algorithm to generate a new prediction of the measure of treatment pain for the at least one step; and
generate a user-perceptible output indicative of the new prediction.

## Patentansprüche

1. Computerimplementiertes Verfahren (8) zur Verwendung bei der Bereitstellung einer Führung für einen
Zahnarzt in Bezug auf eine Zahnbehandlung, wobei das Verfahren umfasst:
Ausführen eines Vorhersagealgorithmus (10), wobei der Vorhersagealgorithmus mindestens einen trainierten maschinellen Lernalgorithmus umfasst, wobei der Vorhersagealgorithmus konfiguriert ist zum:
Empfangen (12) von Eingabevariablen, die mindestens Folgendes umfassen: einen vom Patienten benötigten Behandlungstyp, ausgewählt aus einer vordefinierten Menge möglicher Behandlungstypen;
Erzeugen (14) eines Ausgabevorhersagedatensatzes, der eine vorhergesagte Messung von Behandlungsschmerzen für jeden einer Reihe von Schritten umfasst, die durch den Eingabetyp der Behandlung umfasst sind; und
Ausführen eines Führungsalgorithmus (20), wobei der Führungsalgorithmus konfiguriert ist zum:
Zugreifen (22) auf den Vorhersagedatensatz;
Erzeugen (24) von Führungsinformationen, die die vorhergesagten Behandlungsschmerzen für mindestens eine Teilmenge der Reihe von Behandlungsschritten anzeigen;
Steuern (26) einer Benutzerschnittstelle, um mindestens eine für den Benutzer wahrnehmbare Ausgabe zu erzeugen, die die Steuerinformationen anzeigt;
wobei der Vorhersagealgorithmus einen maschinellen Lernalgorithmus umfasst, der unter Verwendung einer Trainingsdatenbank vorab trainiert wurde, die jeweilige Dateneinträge für jeden einer Vielzahl von früheren Besuchen einer Vielzahl verschiedener Patienten bei einer Reihe verschiedener Zahnbehandlungsanbieter umfasst, und wobei jeder Besuchseintrag mindestens eine Angabe von Folgendem einschließt:
- eine Art der Behandlung, die verabreicht wird; und
- ein Maß, das indikativ für die Schmerzempfindung während der Behandlung ist, für jeden einer Reihe von Schritten, die die Behandlung umfasst.

2. Verfahren nach Anspruch 1, wobei der Vorhersagealgorithmus konfiguriert ist, um Eingabevariablen zu empfangen, die weiter einschließen: einen vom Zahnarzt zu implementierenden Behandlungsstil, ausgewählt aus einem vordefinierten Satz möglicher Behandlungsstile.

3. Verfahren nach Anspruch 1 oder 2, wobei das Verfahren das Steuern der Benutzerschnittstelle umfasst, um eine geordnete Sequenz von für den Benutzer wahrnehmbaren Ausgaben zu erzeugen, die jeweils indikativ für die vorhergesagten Behandlungsschmerzen für einen der Behandlungsschritte der Serie sind.

4. Verfahren nach einem der Ansprüche 1-3, weiter umfassend:
Zugreifen auf eine Planungsdatenbank, die Datensätze speichert, die indikativ für geplante Behandlungssitzungen sind, wobei jeder Datensatz mindestens eine Angabe eines Typs einer durchzuführenden Behandlung einschließt;
Empfangen von Informationen, die indikativ für einen vom Zahnarzt zu implementierenden Behandlungsstil sind, und wobei der zu implementierende Behandlungsstil gegebenenfalls auch als Teil jedes Datensatzes in der Planungsdatenbank umfasst ist;
Erzeugen der Eingabevariablen für den Vorhersagealgorithmus basierend auf dem Zugreifen und dem Empfangen.

5. Verfahren nach Anspruch 2 oder einem der Ansprüche 3-4, wenn abhängig von Anspruch 2, wobei jeder Besuchseintrag der Trainingsdatenbank weiter eine Angabe einschließt von:
- einem Behandlungsstil, wobei der Behandlungsstil einer der vordefinierten Behandlungsstile ist.

6. Verfahren nach einem der Ansprüche 1-5,
wobei innerhalb jedes Besuchseintrags die Messung, die die Behandlungsschmerzen für jede der Reihe von Schritten angibt, einer insgesamt gemessenen Schmerzmenge entspricht, wobei die insgesamt gemessene Schmerzmenge einem Produkt aus einem gemessenen Schmerzreaktionsgrad und einer Zeit entspricht, während der der Schmerzreaktionsgrad empfunden wurde, für jede einer oder mehrerer Schmerzreaktions-Episoden jedes Behandlungsschritts;
wobei innerhalb jedes Besuchseintrags die Messung, die die Behandlungsschmerzen für jede der Reihe von Schritten anzeigt, einen maximalen oder Peak-Schmerzgrad umfasst, der während des jeweiligen Behandlungsschritts empfunden wurde; und/oder
wobei innerhalb jedes Besuchseintrags die Messung, die die Behandlungsschmerzen für jede der Reihe von Schritten anzeigt, eine Messung einer maximalen Änderungsrate des Schmerzgrads umfasst, der während jedem der Behandlungsschritte empfunden wurde.

7. Verfahren nach einem der Ansprüche 1-6, weiter umfassend:
Erhalten einer Maßzahl, die indikativ für die geschätzten tatsächlichen Behandlungsschmerzen für mindestens eine Teilmenge der Behandlungsschritte ist;
Vergleichen der geschätzten tatsächlichen Behandlungsschmerzen und der vorhergesagten Behandlungsschmerzen für jede der mindestens einen Teilmenge der Schritte;
Erzeugen einer Feedbackinformation für den Zahnarzt basierend auf dem Vergleich, z. B. indikativ für ein Ergebnis des Vergleichs.

8. Verfahren nach Anspruch 7, wobei die Angabe, die die geschätzten tatsächlichen Behandlungsschmerzen für jeden der mindestens einen Teilschritt der Behandlung anzeigt, in Echtzeit während der Durchführung jedes der Teilschritte der Behandlung durch den Zahnarzt empfangen wird, und wobei der Vergleich und die Erzeugung des Feedbacks in Echtzeit mit dem Empfang der geschätzten tatsächlichen Behandlungsschmerzen für jeden Schritt erzeugt werden.

9. Verfahren nach Anspruch 7 oder 8, wobei das Erhalten eines Maßes, das indikativ für die geschätzten tatsächlichen Behandlungsschmerzen für mindestens eine Teilmenge der Schritte der Behandlung ist, umfasst:
Empfangen eines biologischen Signals für den Betreff während jedes der mindestens Teilschritte der Behandlung von einer mit dem Betreff gekoppelten biologischen Signalerfassungseinrichtung;
Identifizieren physiologischer Reaktionsereignisse in dem biologischen Signal;
Bestimmen des Maßes, das indikativ für geschätzte tatsächliche Behandlungsschmerzen für jeden der Schritte der Behandlung ist, basierend auf den für diesen Schritt identifizierten physiologischen Reaktionsereignissen.

10. Verfahren nach einem der Ansprüche 1-9, weiter umfassend:
Empfangen von Daten, die indikativ für Bewegungs- und/oder Kraftmuster eines zahnärztlichen Werkzeugs während mindestens eines Schritts der Behandlung sind; und
Anwenden eines Behandlungsstil-Analysatoralgorithmus, der konfiguriert ist, um basierend auf den Bewegungs- und/oder Kraftmustern einen damit assoziierten Behandlungsstil zu schätzen.

11. Verfahren nach Anspruch 10, weiter umfassend
erneutes Ausführen des Vorhersagealgorithmus unter Verwendung des durch den Behandlungsstil-Analysator-Algorithmus geschätzten Behandlungsstils, um eine neue Vorhersage des Maßes der Behandlungsschmerzen für den mindestens einen Schritt zu erzeugen; und
Erzeugen einer für den Benutzer wahrnehmbaren Ausgabe, die die neue Vorhersage indikativ angibt.

12. Computerprogrammprodukt, umfassend Codierungsmittel, die so konfiguriert ist, dass sie bei Ausführung auf einem Prozessor den Prozessor veranlasst, ein Verfahren nach einem der Ansprüche 1-11 durchzuführen.

13. Verarbeitungseinheit (32), umfassend:
eine Eingabe/Ausgabe; und
einen oder mehrere Prozessoren, die zu Folgendem geeignet sind:
Ausführen eines Vorhersagealgorithmus (34), wobei der Vorhersagealgorithmus konfiguriert ist zum:
Empfangen von Eingabevariablen, die mindestens Folgendes umfassen: einen vom Patienten (38) benötigten Behandlungstyp, ausgewählt aus einer vordefinierten Menge möglicher Behandlungstypen; und
Erzeugen eines Ausgabevorhersagedatensatzes (42), der eine vorhergesagte Messung von Behandlungsschmerzen für jeden einer Reihe von Schritten umfasst, die durch den Eingabetyp der Behandlung umfasst sind; und
Ausführen eines Führungsalgorithmus (20), wobei der Führungsalgorithmus konfiguriert ist zum:
Zugreifen auf den Vorhersagedatensatz;
Erzeugen von Führungsinformationen (52), die die vorhergesagten Behandlungsschmerzen für mindestens eine Teilmenge der Reihe von Behandlungsschritten anzeigen;
Steuern einer Benutzerschnittstelle (62), um mindestens eine für den Benutzer wahrnehmbare Ausgabe (64) zu erzeugen, die die Steuerinformationen anzeigt;
wobei der Vorhersagealgorithmus einen maschinellen Lernalgorithmus umfasst, der unter Verwendung einer Trainingsdatenbank vorab trainiert wurde, die jeweilige Dateneinträge für jeden einer Vielzahl von früheren Besuchen einer Vielzahl verschiedener Patienten bei einer Reihe verschiedener Zahnbehandlungsanbieter umfasst, und wobei jeder Besuchseintrag mindestens eine Angabe von Folgendem einschließt:
- eine Art der Behandlung, die verabreicht wird; und
- ein Maß, das indikativ für die Schmerzempfindung während der Behandlung ist, für jeden einer Reihe von Schritten, die die Behandlung umfasst.

14. Verarbeitungseinheit nach Anspruch 13, wobei der Vorhersagealgorithmus konfiguriert ist, um Eingabevariablen zu empfangen, die weiter einschließen: einen vom Zahnarzt zu implementierenden Behandlungsstil, ausgewählt aus einem vordefinierten Satz möglicher Behandlungsstile.

15. Verarbeitungseinheit nach Anspruch 13 oder 14, wobei der eine oder die mehreren Prozessoren weiter dazu geeignet sind, die Benutzerschnittstelle zu steuern, um eine geordnete Sequenz von für den Benutzer wahrnehmbaren Ausgaben zu erzeugen, die jeweils indikativ für die vorhergesagten Behandlungsschmerzen für einen der Behandlungsschritte der Serie sind.

16. Verarbeitungseinheit nach einem der Ansprüche 13-15, wobei ein oder mehrere Prozessoren weiter geeignet sind zum:
Zugreifen auf eine Planungsdatenbank, die Datensätze speichert, die indikativ für geplante Behandlungssitzungen sind, wobei jeder Datensatz mindestens eine Angabe eines Typs einer durchzuführenden Behandlung einschließt;
Empfangen von Informationen, die indikativ für einen vom Zahnarzt zu implementierenden Behandlungsstil sind, und wobei der zu implementierende Behandlungsstil gegebenenfalls auch als Teil jedes Datensatzes in der Planungsdatenbank umfasst ist;
Erzeugen der Eingabevariablen für den Vorhersagealgorithmus basierend auf dem Zugreifen und dem Empfangen.

17. Verarbeitungseinheit nach Anspruch 14 oder einem der Ansprüche 15-16, wenn abhängig von Anspruch 14, wobei jeder Besuchseintrag der Trainingsdatenbank weiter eine Angabe einschließt von:
- einem Behandlungsstil, wobei der Behandlungsstil einer der vordefinierten Behandlungsstile ist.

18. Verarbeitungseinheit nach einem der Ansprüche 13-17,
wobei innerhalb jedes Besuchseintrags die Messung, die die Behandlungsschmerzen für jede der Reihe von Schritten angibt, einer insgesamt gemessenen Schmerzmenge entspricht, wobei die insgesamt gemessene Schmerzmenge einem Produkt aus einem gemessenen Schmerzreaktionsgrad und einer Zeit entspricht, während der der Schmerzreaktionsgrad empfunden wurde, für jede einer oder mehrerer Schmerzreaktions-Episoden jedes Behandlungsschritts;
wobei innerhalb jedes Besuchseintrags die Messung, die die Behandlungsschmerzen für jede der Reihe von Schritten anzeigt, einen maximalen oder Peak-Schmerzgrad umfasst, der während des jeweiligen Behandlungsschritts empfunden wurde; und/oder
wobei innerhalb jedes Besuchseintrags die Messung, die die Behandlungsschmerzen für jede der Reihe von Schritten anzeigt, eine Messung einer maximalen Änderungsrate des Schmerzgrads umfasst, der während jedem der Behandlungsschritte empfunden wurde.

19. Verarbeitungseinheit nach einem der Ansprüche 13-18, wobei der eine oder mehrere Prozessoren weiter geeignet sind zum:
Erhalten einer Maßzahl, die indikativ für die geschätzten tatsächlichen Behandlungsschmerzen für mindestens eine Teilmenge der Behandlungsschritte ist;
Vergleichen der geschätzten tatsächlichen Behandlungsschmerzen und der vorhergesagten Behandlungsschmerzen für jeden der mindestens einen Teilmenge der Schritte;
Erzeugen einer Feedbackinformation für den Zahnarzt basierend auf dem Vergleich, z. B. indikativ für ein Ergebnis des Vergleichs.

20. Verarbeitungseinheit nach Anspruch 19, wobei die Angabe, die die geschätzten tatsächlichen Behandlungsschmerzen für jeden der mindestens einen Teilschritt der Behandlung anzeigt, in Echtzeit während der Durchführung jedes der Teilschritte der Behandlung durch den Zahnarzt empfangen wird, und wobei der Vergleich und die Erzeugung des Feedbacks in Echtzeit mit dem Empfang der geschätzten tatsächlichen Behandlungsschmerzen für jeden Schritt erzeugt werden.

21. Verarbeitungseinheit nach Anspruch 19 oder 20, wobei das Erhalten eines Maßes, das indikativ für die geschätzten tatsächlichen Behandlungsschmerzen für mindestens eine Teilmenge der Schritte der Behandlung ist, umfasst:
Empfangen eines biologischen Signals für den Betreff während jedes der mindestens Teilschritte der Behandlung von einer mit dem Betreff gekoppelten biologischen Signalerfassungseinrichtung;
Identifizieren physiologischer Reaktionsereignisse in dem biologischen Signal;
Bestimmen des Maßes, das indikativ für geschätzte tatsächliche Behandlungsschmerzen für jeden der Schritte der Behandlung ist, basierend auf den für diesen Schritt identifizierten physiologischen Reaktionsereignissen.

22. Verarbeitungseinheit nach einem der Ansprüche 13-21, wobei der eine oder mehrere Prozessoren weiter geeignet sind zum:
Empfangen von Daten, die indikativ für Bewegungs- und/oder Kraftmuster eines zahnärztlichen Werkzeugs während mindestens eines Schritts der Behandlung sind; und
Anwenden eines Behandlungsstil-Analysatoralgorithmus, der konfiguriert ist, um basierend auf den Bewegungs- und/oder Kraftmustern einen damit assoziierten Behandlungsstil zu schätzen.

23. Verarbeitungseinheit nach Anspruch 22, wobei der eine oder mehrere Prozessoren weiter geeignet sind zum:
erneuten Ausführen des Vorhersagealgorithmus unter Verwendung des durch den Behandlungsstil-Analysator-Algorithmus geschätzten Behandlungsstils, um eine neue Vorhersage des Maßes der Behandlungsschmerzen für den mindestens einen Schritt zu erzeugen; und
Erzeugen einer für den Benutzer wahrnehmbaren Ausgabe, die die neue Vorhersage indikativ angibt.

## Revendications

1. Procédé mis en œuvre par ordinateur (8) destiné à être utilisé pour fournir des conseils à un praticien
dentaire en ce qui concerne un traitement dentaire, le procédé comprenant :
l'exécution d'un algorithme de prédiction (10), l'algorithme de prédiction comprenant au moins un algorithme d'apprentissage automatique entraîné, dans lequel l'algorithme de prédiction est configuré pour :
recevoir (12) des variables d'entrée comprenant au moins : un type de traitement requis par le patient, sélectionné dans un ensemble prédéfini de types de traitement possibles ;
générer (14) un jeu de données de prédiction de sortie comprenant une mesure prédite de douleur de traitement pour chaque étape d'une série d'étapes constituées par le type de traitement d'entrée ; et
l'exécution d'un algorithme de conseils (20), l'algorithme de conseils étant configuré pour :
avoir accès (22) au jeu de données de prédiction ;
générer (24) des informations de conseils indiquant la douleur de traitement prédite pour au moins un sous-ensemble de la série d'étapes de traitement ;
commander (26) une interface utilisateur pour générer au moins une sortie perceptible par l'utilisateur indiquant les informations de conseils ;
dans lequel l'algorithme de prédiction comprend un algorithme d'apprentissage automatique pré-entraîné utilisant une base de données d'entraînement comprenant des entrées de données respectives pour chaque antérieure d'une pluralité de visites antérieures par une pluralité de patients différents auprès d'un ensemble de différents prestataires de traitement dentaire, et dans lequel chaque entrée de visite inclut au moins une indication :
- d'un type de traitement administré ; et
- d'une mesure indiquant la douleur de traitement pour chaque étape d'une série d'étapes comprises dans le traitement.

2. Procédé selon la revendication 1, dans lequel l'algorithme de prédiction est configuré pour recevoir des variables d'entrée qui incluent en outre : un style de traitement à mettre en œuvre par le praticien dentaire, sélectionné dans un ensemble prédéfini de styles de traitement possibles.

3. Procédé selon la revendication 1 ou 2, le procédé comprenant la commande de l'interface utilisateur pour générer une séquence ordonnée de sorties perceptibles par l'utilisateur, chacune indiquant la douleur de traitement prédite pour une étape de traitement de la série d'étapes de traitement.

4. Procédé selon l'une quelconque des revendications 1-3, comprenant en outre :
l'accès à une base de données de planification stockant des enregistrements indiquant des sessions de traitement programmées, chaque enregistrement incluant au moins une indication d'un type de traitement à effectuer ;
la réception d'informations indiquant un style de traitement à mettre en œuvre par le praticien dentaire, et facultativement dans lequel le style de traitement à mettre en œuvre est également compris dans le cadre de chaque enregistrement de données dans la base de données de planification ;
la génération des variables d'entrée pour l'algorithme de prédiction sur la base desdites étapes d'accès et de réception.

5. Procédé selon la revendication 2 ou l'une quelconque des revendications 3-4 lorsqu'elles dépendent de la revendication 2, dans lequel chaque entrée de visite de la base de données d'entraînement inclut en outre une indication :
- d'un style de traitement, dans lequel le style de traitement est un style de traitement des ensembles prédéfinis de styles de traitement.

6. Procédé selon l'une quelconque des revendications 1-5,
dans lequel, dans chaque entrée de visite, la mesure indiquant la douleur de traitement pour chaque étape de la série d'étapes correspond à une quantité totale de douleur mesurée, la quantité totale de douleur mesurée correspondant à un produit d'un niveau de réponse à la douleur mesuré et d'un temps pendant lequel le niveau de réponse à la douleur a été ressenti, pour chacune d'une ou de plusieurs épisodes de réponse à la douleur de chaque étape de traitement ;
dans lequel, dans chaque entrée de visite, la mesure indiquant la douleur de traitement pour chaque étape de la série d'étapes comprend un niveau de douleur maximal ou de pointe ressenti pendant l'étape de traitement respective ; et/ou
dans lequel, dans chaque entrée de visite, la mesure indiquant la douleur de traitement pour chaque étape de la série d'étapes comprend une mesure d'un taux maximal de changement du niveau de douleur ressenti pendant chacune des étapes de traitement.

7. Procédé selon l'une quelconque des revendications 1-6, comprenant en outre :
l'obtention d'une mesure indiquant la douleur de traitement réelle estimée pour au moins un sous-ensemble des étapes du traitement ;
la comparaison, pour chaque étape du au moins un sous-ensemble d'étapes, de la douleur de traitement réelle estimée et de la douleur de traitement prédite ;
la génération d'informations de rétroaction pour le praticien dentaire sur la base de la comparaison, par ex. indiquant un résultat de la comparaison.

8. Procédé selon la revendication 7, dans lequel la mesure indiquant la douleur de traitement réelle estimée pour chaque étape dudit au moins un sous-ensemble d'étapes du traitement est reçue en temps réel pendant l'exécution de chaque étape du sous-ensemble des étapes du traitement par le praticien dentaire, et dans lequel la comparaison et la génération de rétroaction sont générées en temps réel avec la réception de la douleur de traitement réelle estimée pour chaque étape.

9. Procédé selon la revendication 7 ou 8, dans lequel l'obtention d'une mesure indiquant la douleur de traitement réelle estimée pour au moins un sous-ensemble des étapes du traitement comprend :
la réception, pendant chaque étape du au moins un sous-ensemble d'étapes du traitement, d'un signal biologique pour le sujet provenant d'un appareil de détection de signal biologique couplé au sujet ;
l'identification d'événements de réponse physiologique dans le signal biologique ;
la détermination de la mesure indiquant la douleur de traitement réelle estimée pour chaque dite étape du traitement sur la base des événements de réponse physiologique identifiés pour cette étape.

10. Procédé selon l'une quelconque des revendications 1-9, comprenant en outre :
la réception de données indiquant un mouvement et/ou des motifs de force d'un outil dentaire pendant au moins une étape du traitement ; et
l'application d'un algorithme d'analyseur de style de traitement configuré pour estimer, sur la base du mouvement et/ou de motifs de force, un style de traitement associé à celui-ci.

11. Procédé selon la revendication 10, comprenant en outre
la réexécuttion de l'algorithme de prédiction utilisant le style de traitement estimé par l'algorithme d'analyseur de style de traitement pour générer une nouvelle prédiction de la mesure de la douleur de traitement pour la au moins une étape ; et
la génération d'une sortie perceptible par l'utilisateur indiquant la nouvelle prédiction.

12. Produit de programme informatique comprenant des moyens de code configurés, lorsqu'ils sont exécutés sur un processeur, pour amener le processeur à réaliser un procédé selon l'une quelconque des revendications 1-11.

13. Unité de traitement (32), comprenant :
une entrée/sortie ; et
un ou plusieurs processeurs, adaptés pour :
exécuter un algorithme de prédiction (34), l'algorithme de prédiction étant configuré pour :
recevoir des variables d'entrée comprenant au moins : un type de traitement requis par le patient (38), sélectionné dans un ensemble prédéfini de types de traitement possibles, et
générer un jeu de données de prédiction de sortie (42) comprenant une mesure prédite de douleur de traitement pour chaque étape d'une série d'étapes constituées par le type de traitement d'entrée ; et
exécuter un algorithme de conseils (20), l'algorithme de conseils étant configuré pour :
avoir accès au jeu de données de prédiction ;
générer des informations de conseils (52) indiquant la douleur de traitement prédite pour au moins un sous-ensemble de la série d'étapes de traitement ;
commander une interface utilisateur (62) pour générer au moins une sortie (64) perceptible par l'utilisateur indiquant les informations de conseils ;
dans laquelle l'algorithme de prédiction comprend un algorithme d'apprentissage automatique pré-entraîné utilisant une base de données d'entraînement comprenant des entrées de données respectives pour chaque visite antérieure d'une pluralité de visites antérieures par une pluralité de différents patients auprès d'un ensemble de différents prestataires de traitement dentaire, et dans laquelle chaque entrée de visite inclut au moins une indication :
- d'un type de traitement administré ; et
- d'une mesure indiquant la douleur de traitement pour chaque étape d'une série d'étapes comprises dans le traitement.

14. Unité de traitement selon la revendication 13, dans laquelle l'algorithme de prédiction est configuré pour recevoir des variables d'entrée qui incluent en outre : un style de traitement à mettre en œuvre par le praticien dentaire, sélectionné dans un ensemble prédéfini de styles de traitement possibles.

15. Unité de traitement selon la revendication 13 ou 14, les un ou plusieurs processeurs étant en outre adaptés pour commander l'interface utilisateur pour générer une séquence ordonnée de sorties perceptibles par l'utilisateur, chacune indiquant la douleur de traitement prédite pour une étape de traitement de la série d'étapes de traitement.

16. Unité de traitement selon l'une quelconque des revendications 13-15, les un ou plusieurs processeurs étant en outre adaptés pour :
avoir accès à une base de données de planification stockant des enregistrements indiquant des sessions de traitement programmées, chaque enregistrement incluant au moins une indication d'un type de traitement à effectuer ;
recevoir des informations indiquant un style de traitement à mettre en œuvre par le praticien dentaire et, facultativement, dans laquelle le style de traitement à mettre en œuvre est également compris dans le cadre de chaque enregistrement de données dans la base de données de planification ;
générer les variables d'entrée pour l'algorithme de prédiction sur la base desdites étapes d'accès et de réception.

17. Unité de traitement selon la revendication 14 ou l'une quelconque des revendications 15-16 lorsqu'elles dépendent de la revendication 14, dans laquelle chaque entrée de visite de la base de données d'entraînement inclut en outre une indication :
- d'un style de traitement, dans laquelle le style de traitement est un style de traitement des ensembles prédéfinis de styles de traitement.

18. Unité de traitement selon l'une quelconque des revendications 13-17,
dans laquelle, dans chaque entrée de visite, la mesure indiquant la douleur de traitement pour chaque étape de la série d'étapes correspond à une quantité totale de douleur mesurée, la quantité totale de douleur mesurée correspondant à un produit d'un niveau de réponse à la douleur mesuré et d'un temps pendant lequel le niveau de réponse à la douleur a été ressenti, pour chacune d'une ou de plusieurs épisodes de réponse à la douleur de chaque étape de traitement ;
dans laquelle, dans chaque entrée de visite, la mesure indiquant la douleur de traitement pour chaque étape de la série d'étapes comprend un niveau de douleur maximal ou de pointe ressenti pendant l'étape de traitement respective ; et/ou
dans laquelle, dans chaque entrée de visite, la mesure indiquant la douleur de traitement pour chaque étape de la série d'étapes comprend une mesure d'un taux maximal de changement du niveau de douleur ressenti pendant chacune des étapes de traitement.

19. Unité de traitement selon l'une quelconque des revendications 13-18, dans laquelle les un ou plusieurs processeurs sont en outre adaptés pour :
obtenir une mesure indiquant la douleur de traitement réelle estimée pour au moins un sous-ensemble des étapes du traitement ;
comparer pour chaque étape du au moins un sous-ensemble d'étapes la douleur de traitement réelle estimée et la douleur de traitement prédite ;
générer des informations de rétroaction pour le praticien dentaire sur la base de la comparaison, par ex. indiquant un résultat de la comparaison.

20. Unité de traitement selon la revendication 19, dans laquelle la mesure indiquant la douleur de traitement réelle estimée pour chaque étape dudit au moins un sous-ensemble d'étapes du traitement est reçue en temps réel pendant l'exécution de chaque étape du sous-ensembles des étapes du traitement par le praticien dentaire, et dans laquelle la comparaison et la génération de rétroaction sont générées en temps réel avec la réception de la douleur de traitement réelle estimée pour chaque étape.

21. Unité de traitement selon la revendication 19 ou 20, dans laquelle l'obtention d'une mesure indiquant la douleur de traitement réelle estimée pour au moins un sous-ensemble des étapes du traitement comprend :
la réception, pendant chaque étape du au moins un sous-ensemble d'étapes du traitement, d'un signal biologique pour le sujet provenant d'un appareil de détection de signal biologique couplé au sujet ;
l'identification d'événements de réponse physiologique dans le signal biologique ;
la détermination de la mesure indiquant la douleur de traitement réelle estimée pour chaque dite étape du traitement sur la base des événements de réponse physiologique identifiés pour cette étape.

22. Unité de traitement selon l'une quelconque des revendications 13-21, les un ou plusieurs processeurs étant en outre adaptés pour :
recevoir des données indiquant un mouvement et/ou des motifs de force d'un outil dentaire pendant au moins une étape du traitement ; et
appliquer un algorithme d'analyseur de style de traitement configuré pour estimer, sur la base du mouvement et/ou de motifs de force, un style de traitement associé à celui-ci.

23. Unité de traitement selon la revendication 22, les un ou plusieurs processeurs étant en outre adaptés pour :
réexécuter l'algorithme de prédiction utilisant le style de traitement estimé par l'algorithme d'analyseur de style de traitement pour générer une nouvelle prédiction de la mesure de la douleur de traitement pour la au moins une étape ; et
générer une sortie perceptible par l'utilisateur indiquant la nouvelle prédiction.
